# EUROPÄISCHE PATENTANMELDUNG

(11) **EP 0 600 459 A1**
(43) Veröffentlichungstag der Anmeldung: **08.06.1994**
(21) Anmeldenummer: 93119341.1
(22) Anmeldetag: 01.12.1993
(51) Int. Cl.: B65D 30/02, B65D 65/38, A47C 31/10, A47C 21/06, A47G 9/02

(54) **Verwendung eines Spinnvlieses aus Polyethylenfasern für verschliessbare Behälter zur Aufnahme von mit Allergenen kontaminierten Gegenständen**

(30) Priorität: 04.12.1992 DE 4240829
(71) Anmelder: Siewert, Ronald-R., c/o INNOV-ALL PHARMA VERTRIEBS GmbH & CO. KG, D-40231 Düsseldorf (DE)
(72) Erfinder: Siewert, Ronald-R., c/o INNOV-ALL PHARMA VERTRIEBS GmbH & CO. KG, D-40231 Düsseldorf (DE)
(74) Vertreter: Meyers, Hans-Wilhelm, Dr.Dipl.-Chem.

(57) **Zusammenfassung**

Verwendung eines Spinnvlieses aus Polyethylenfasern für verschließbare Behälter zur Aufnahme von mit Allergenen, wie Hausstaubmilben oder deren allergene Ausscheidungen, kontaminierten Gegenstände, wobei der verschließbare Behälter die kontaminierten Gegenstände umschließt. Ein Behälter zur erfindungsgemäßen Verwendung weist einen Verschluß (5) auf, wobei der Verschluß aus zwei Hälften (5a,5b) aufgebaut ist, die an einer umgebogenen, eine Öffnung im Behälter (1) bildenden Kante (15,25) jeweils an dem Behälter (1) befestigt sind und wobei zum allergendichten Verschließen des Behälters (1) am Verschluß 5 mindestens eine Bahn (30) und/oder (31), die mit dem Verschluß (5) überlappend angeordnet ist (sind).

## Beschreibung

Gegenstand der vorliegenden Erfindung ist eine Verwendung gemäß Anspruch 1 sowie ein Behälter gemäß Anspruch 7.

Für Patienten mit einer Hausstaubmilben-Allergie, einem Milben-Asthma oder einem atopischen Ekzem ist das Bett die Hauptallergenquelle. Zur Ausschaltung dieser Allergenquelle werden Akarizide (milbenabtötende Mittel), die nur auf der Oberfläche von textilen Gegenständen angewendet werden, eingesetzt. Diese zeigen jedoch im Bettenbereich den gewünschten Erfolg nicht im hinreichenden Maße, denn der textile Aufbau beispielsweise von Matratzen ist so komplex, daß es nicht möglich ist, die in der Tiefe lebenden Hausstaubmilben innerhalb kurzer Zeit zu vernichten. Erst wenn die Milben beseitigt sind, wird die Produktion allergenhaltiger Stoffe, wie Exkremente der Milben, unterbunden. Ebenso lassen sich allergenhaltige Partikel, die bereits die Matratzen oder anderes Bettzeug infiltriert haben, kaum vollständig entfernen. So haben klinische Prüfungen ergeben, daß es Monate oder gar Jahre dauern kann, um Allergene aus benutzten Matratzen zu entfernen.

Aus der Sicht des behandelnden Arztes und vor allem auch des Allergikers muß die Allergenquelle möglichst schnell und effektiv blockiert werden. Nur so wird die Ursache für die allergische Reaktion ausgeschaltet. Die Anwendung akarizider Mittel im Bettenbereich ist nicht unbedenklich, da nicht sicher abgeschätzt werden kann, ob es bei Daueranwendung akarizider oder chemischer Mittel zu weiteren Irritationen der Atemwege kommen kann.

Es ist ebenfalls versucht worden durch Laminate, die aus Baumwollgewebe mit darunterliegendem, undurchlässigem Material eine Barriere für allergene Stoffe, aus Bettzeug stammen, zu schaffen. Nachteilig daran ist jedoch, daß sich die Laminate durch Druck, beispielsweise wie ihn eine liegende Person ausübt oder durch mehrfaches Waschen, lösen und nach kurzem Gebrauch bereits ihre Funktion nicht mehr hinreichend erfüllen können. Auch Gewebe mit einer Membran, z. B. aus Teflon, wie sie in der Textilindustrie als Goretex im Gebrauch sind, sind nicht unbedenklich, da sie bei der Entsorgung große Probleme aufwerfen.

Das deutsche Gebrauchsmuster G 92 09 415.5 U1 betrifft eine Verpackung aus einem Kunststoffvlies oder -filz. Die dort beschriebene Verpackung wird als Behältnis für Gegenstände aller Art für Transport und Lagerung, insbesondere zum Schutz der verpackten Gegenstände gegen Verschmutzung und Beschädigung beschrieben. Als Materialien zur Herstellung der dort beschriebenen Verpackungen kommen Vliese aus thermoplastischen Fasern in Frage wie Polyester, Polypropylene und Polyamide. Die dort beschriebenen Verpackungsbehältnisse sind insbesondere deshalb vorteilhaft, da sie sich zum Nichtgebrauch flach zusammenlegen lassen.

Das deutsche Gebrauchsmuster G 83 37 358.6 U1 betrifft eine abstreifbare Hülle zum Überziehen von dreidimensionalen Gegenständen aus einem Stück Gewebe oder Faservlies, wobei an den Endzipfeln der Hülle von wenigstens einer der beiden entgegengesetzten Seiten eine in der Nähe der beiden nicht zusammengefalteten Seiten genähte Zusammenfaltung dieser Zipfel aufweist.

Das Gebrauchsmuster G 88 06 808.0 U1 betrifft einen Kleider- und Pelzschutzsack aus synthetischen Vliesstoffen. Dieser Sack ist zur Aufnahme von Kleider und Filze gedacht und soll die Nachteile, die durch natürliche Gewebe hergestellten Kleidersäcke vermeiden.

Das der Erfindung zugrunde liegende Problem besteht in der Bereitstellung eines Mittels zur wirksamen Blockade der Freisetzung von Allergenen aus damit belasteten Gegenständen, das sicher und schnell wirkt und bei der Entsorgung die Umwelt nicht ungebührlich belastet.

Überraschenderweise wird das der Erfindung zugrunde liegende Problem gelöst durch eine neuartige Verwendung eines Spinnvlieses aus Polyethylenfasern für verschließbare Behälter mit den Merkmalen des Patentanspruchs 1. Bevorzugte Ausführungsformen der erfindungsgemäßen Verwendung werden in den daran anschließenden Unteransprüchen 2 bis 6 beschrieben. Anspruch 7 betrifft eine bevorzugte Ausführung eines Behälters, der geeignet ist, erfindungsgemäß verwendet zu werden.

Der durch die erfindungsgemäße Verwendung des Spinnvlieses aus Polyethylenfasern hergestellte Behälter zur Aufnahme von mit Allergenen kontaminierten Gegenständen, wie Hausstaubmilben, Allergenen dieser Milben, wie Exkremente, umschließt die kontaminierten Gegenstände. Vorzugsweise ist der Behälter flexibel, wodurch eine gute Anpassung an den zu umschließenden Gegenstand erreicht wird.

Der Behälter weist vorzugsweise ungefähr die Form des aufzunehmenden Gegenstandes auf. Als kontaminierte Gegenstände im Sinne der Erfindung sind insbesondere Materialien zu verstehen, wie sie im Bettbereich verwendet werden also Kissen, Matratzen, Ober- und Unterbetten, Decken etc, aber auch Polster in Polstermöbeln.

Der Verschluß des verschließbaren Behälters besteht vorzugsweise aus einem leicht zu öffnenden oder schließenden Verschluß, der an mindestens einer Seite des Behälters angeordnet ist. Der Verschluß kann beispielsweise aus einem dichtschließenden Klett-, Reiß- oder einem Gleitverschluß bestehen. Vorzugsweise weist der Gleitverschluß einen zweifachen Falzverschluß auf.

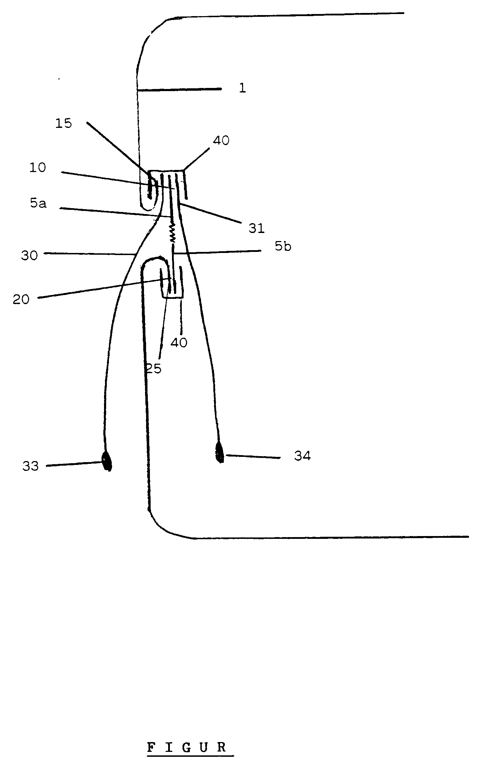

Die Figur zeigt eine Schnittdarstellung des erfindungsgemäß zu verwendenden Behälters im Bereich des Verschlusses. Die bevorzugte Ausführungsform des Behälters 1 ist an einer Seite mit einem Reiß- oder Gleitverschluß 5 versehen. Um einen allergendichten Verschluß des Behälters 1 zu erreichen, wird der Verschluß 5 an den Nahtstellen 10 und 20, die den Verschluß 5 mit dem Behälter 1 verbinden, befestigt. Die die Öffnung bildenden Kanten 15 und 25 des Behälters 1 werden vorzugsweise umgebogen und mit den jeweiligen Verschlußhälften 5a und 5b fest verbunden, vorzugsweise verschweißt. In einer bevorzugten Variante wird dann zwischen der umgebogenen Kante 15 und der Verschlußhälfte 5a eine Bahn 31 angeordnet, die mit der Kante 15 und der Verschlußhälfte 5a verbunden wird. In einer anderen Ausführungsform wird eine Bahn 31 an der zur Innenseite des Behälters 1 weisenden Seite der Verschlußhälfte 5a angeordnet und ebenfalls vorzugsweise durch Verschweißen verbunden. Die Bahnen 30 und 31 sind vorzugsweise aus dem gleichen oder einem sehr ähnlichen Material wie dasjenige, des Behälters 1 gefertigt.

Die Bahn 30 sorgt durch Überlappung mit dem Verschluß 5 und der äußeren Begrenzung des Behälters 1 für eine Abdichtung, ebenso wie die Bahn 31 eine Abdichtung zum inneren Bereich des Behälters bewirkt. Eine besonders gute Abschirmung der Allergene, die sich im Behälter sammeln, läßt sich durch gleichzeitige Verwendung der Bahnen 30 und 31 erzielen, jedoch werden hinreichende Ergebnisse auch mit der Verwendung jeweils einer der Bahnen 30 oder 31 erreicht. Vorzugsweise sind die Bahnen 30, 31 an der der Verschweißungsstelle gegenüberliegenden Stelle mit Verstärkungen versehen, um eine Beschädigung der Kante 33, 34 zu verhindern.

In einer weiteren bevorzugten Ausgestaltung des Behälters 1 sind die Nahtstellen 10 und 20 noch mit einem Abdichtungsband 40 versehen.

Falls mehrere Bahnen des Spinnvlieses verschiedener Größen zum Zusammenbau des Behälters erfindungsgemäß verwendet werden, wie beispielsweise ein Behälter zur Aufnahme einer Matratze, werden die unterschiedlichen Bahnen an ihrer Stoßkante, vorzugsweise dichtschließend, miteinander verbunden. Dies kann z.B. durch Schweißen oder Verkleben erfolgen. Wenn zwei aneinander stoßende Bahnen vernäht werden oder aber die Verschlüsse an der entsprechenden Stelle an den Behälter genäht werden müssen, werden die durch die Nähnadel entstehenden Löcher vorzugsweise durch Dichtungsmassen wie Silikon oder hautverträgliche Kleber abgedichtet.

So schaffen die z.B durch die erfindungsgemäße Verwendung hergestellten Behälter, insbesondere als Überzüge für Matratzen und Betten, eine sofortige Allergenblockade, weil die Allergene nicht mehr nach außen dringen können. Umgekehrt können weder Milben noch Hautschuppen, die als Nahrung für die Hausstaubmilben dienen, in das Innere von Matratzen und Betten vordringen.

Vorteilhaft wirkt sich bei dem durch die erfindungsgemäße Verwendung hergestellten Behälter aus einem Spinnvlies von Polyethylen-Fasern aus, daß dieser Behälter wasserdampfdurchlässig ist. Dadurch wird verhindert, daß der Patient von dem während des Schlafes abgegebenen Schweiß beeinträchtigt wird, der sich auf dem Überzug sammeln würde, wenn dieser nicht wasserdampfdurchlässig wäre.

Zum Aufbau des Behälters wird erfindungsgemäß vorzugsweise ein Material verwendet, das zu ca. 100 % aus Polyethylen, welches selbst allergologisch unbedenklich ist, besteht. Das Polyethylen weist einen Schmelzpunkt von etwa 135°C und einen Flammpunkt von etwa 330 bis 365°C auf. Die Dichte beträgt vorzugsweise 0,955 g/cm³ und enthält ca. 0,1 Gew.-% flüchtige Bestandteile bei einer Temperatur von 250°C. Das Material ist chemisch beständig und erst oberhalb von 250 bis 300°C hitzeempfindlich. Das Material ist handelsüblich unter der Bezeichnung TYVEK®, Tyvek®PE, Tyvek Saranex® oder Barricade® der Firma Du Pont de Nemours and Company.

In einem an sich bekannten Verfahren werden Polyethylen-Endlosfasern in großer Anzahl in mehreren Lagen, insbesondere sieben Lagen, zu einem Spinnvlies (TYVEK) verarbeitet, das anschließend durch Druck und Wärme verfestigt wird. Das Material ist frei von Bindemitteln, Weißmachern, Füllstoffen, Farbstoffen und anderen Zusätzen. Das Material, aus dem die durch die erfindungsgemäße Verwendung hergestellten Behälter gefertigt werden, ist reißfest, zugfest und abriebfest. Gleichzeitig ist es resistent gegen die meisten organischen und anorganischen Chemikalien, Säuren, Basen und Salzen. Das Material verhält sich chemisch neutral. Vorteilhaft ist das Gewebe, da es dicht, fest und zugleich leicht und anschmiegsam ist und somit für einen hohen Komfort sorgt. Das Material zur Herstellung der Behälter ist voll wasserbeständig. Es verliert diese Eigenschaft weder im trockenen noch im nassen Zustand. Lösungsmittel, Öle und Fette werden unter geringer Quellung und einem geringen Verlust der mechanischen Eigenschaften leicht aufgenommen. Das Material bildet keinen Nährboden für Schimmel und Fäulnis, selbst nicht nach längerer Lagerung in der Erde.

Die durch die erfindungsgemäße Verwendung hergestellten Behälter, insbesondere Überzüge, haben überraschenderweise die Eigenschaft Allergene wie Exkremente der Hausstaubmilbe zurückzuhalten. Das Material, aus dem die Behälter hergestellt werden, sind so dicht, daß selbst Asbestfasern bis zu einer Größe von 0,12 µm zu 99 % nicht passieren können.

Als Allergene, die wirksam mit dem erfindungsgemäßen Behälter zurückgehalten werden können, kommen auch beispielsweise so feine Partikel wie Katzenhaarallergene in Betracht.

Die Behälter, insbesondere Überzüge, aus dem Polyethylen-Spinnvlies sind für Wasserdampf durchlässig. Die Luftdurchlässigkeit beträgt ca. 12 l/min/m².

Das Spinnvlies aus Polyethylen-Fasern ist toxikologisch unbedenklich. Scratch-Tests an der Haut von Menschen und Kaninchen lösten selbst bei mehrmaliger Wiederholung weder Hautirritationen noch Sensibilisierungsreaktionen aus. Bei Augenkontakt tritt lediglich die übliche, durch einen Fremdkörper ausgelöste mechanische Reizung auf.

Polyethylen ist seit langer Zeit im praktischen Gebrauch und hat keinerlei Anhaltspunkte für irritative oder sensibilisierende Eigenschaften ergeben. Allergologisch gilt Polyethylen als inerter Stoff.

Die durch die erfindungsgemäße Verwendung hergestellten Behälter bestehen zu ca. 100 % aus Polyethylen (High Density Polyethylen) und können mithin problemlos wieder zu Polyethylen-Granulat recycelt werden und neuen Verwendungszwecken zugeführt werden. Es kann auch nach anderen an sich bekannten Verfahren zur Polyethylen-Wiedergewinnung verwertet werden oder unter entsprechenden Bedingungen rückstandsfrei zu Wasser und Kohlendioxyd verbrannt werden.

Die Behälter werden in einfacher Weise über die jeweiligen kontaminierten Gegenstände gezogen. So können die Behälter in Form von Überzügen über die Matratzen, das Kopfkissen bzw. Zudecken-Inlett usw. gezogen werden und mit dem Gleit- oder Reißverschluß verschlossen werden. Darüber kann dann die übliche Bettwäsche gezogen werden, die dann in üblichen Zyklen gewechselt werden kann.

Die Behälter, insbesondere Überzüge, halten in vorteilhafter Weise Partikel einer Größe > 0,5 µm zurück. Darüberhinaus ist das Gewebe zum Aufbau der Behälter wasserfest aber durchlässig für Wasserdampf. Die Nähte schließen dicht und lassen keine allergenen Partikel hindurch. Die Behälter sind bei Temperaturen bis zu 30°C mehrfach waschbar. Es ist ausreichend die Überzüge ein bis zweimal pro Jahr mit handelsüblichen Feinwaschmitteln zu reinigen. Nach dem Waschen sollten die Behälter lediglich an der Luft getrocknet werden. Da die Behälter zu nahezu 100 % aus Polyethylen bestehen, sind sie allergologisch unbedenklich. Die Behälter sind frei von Bindemitteln, Weißmachern, Füllstoffen und Farbstoffen und sind reiß-, zug- und abriebfest und gleichzeitig resistent gegen die meisten organischen und anorganischen Chemikalien, Säuren, Basen und Salze. Die Überzüge bzw. das Material verhalten sich chemisch neutral.

## Patentansprüche

1. Verwendung eines Spinnvlieses aus Polyethylenfasern für verschließbare Behälter zur Aufnahme von mit Allergenen, wie Hausstaubmilben oder deren allergene Ausscheidungen, kontaminierten Gegenstände, wobei der verschließbare Behälter die kontaminierten Gegenstände umschließt.

2. Verwendung nach Anspruch 1, wobei der verschließbare Behälter flexibel ist und in Form von Überzügen zur Aufnahme von Bettzeug wie Matratzen, Kissen, Ober- und/oder Unterbetten, Decken vorliegt.

3. Verwendung nach einem der Ansprüche 1 und/oder 2, wobei zum Verschließen des Behälters ein leicht zu öffnender oder schließender Verschluß an mindestens einer Seite des Behälters angeordnet ist und der Verschluß aus einem dichtschließenden Klettverschluß, Reißverschluß oder Gleitverschluß besteht.

4. Verwendung nach mindestens einem der Ansprüche 1 bis 3, wobei der Behälter aus Bahnen des Polyethylen-Spinnvlieses aufgebaut ist, die miteinander dichtschließend wie durch Schweißen und/oder Kleben verbunden sind.

5. Verwendung nach mindestens einem der Ansprüche 1 bis 4, wobei der Gleitverschluß einen mindestens zweifachen Falzverschluß aufweist.

6. Verwendung nach mindestens einem der Ansprüche 1 bis 5, wobei der Behälter an Nahtstellen mit Dichtungsmasse und/oder Kleber abgedichtet ist.

7. Behälter zur Verwendung nach einem der Ansprüche 1 bis 6 mit einem Verschluß (5), wobei der Verschluß aus zwei Hälften (5a, 5b) aufgebaut ist, die an einer umgebogenen, eine Öffnung im Behälter (1) bildenden Kante (15, 25) jeweils an dem Behälter (1) befestigt sind und wobei zum allergendichten Verschließen des Behälters (1) am Verschluß (5) mindestens eine Bahn (30) und/oder (31), die mit dem Verschluß (5) überlappend angeordnet ist (sind).
